(19) Europäisches Patentamt
European Patent Office
Office européen des brevets

(11) Numéro de publication: **0 063 988**
**B1**

(12) **FASCICULE DE BREVET EUROPEEN**

(45) Date de publication du fascicule du brevet: **29.01.86**

(51) Int. Cl.[4]: **A 61 K 39/395,** A 61 K 39/44

(21) Numéro de dépôt: **82400651.4**

(22) Date de dépôt: **09.04.82**

(54) **Médicaments anticancéreux contenant la chaîne A de la ricine associée à un anticorps antimélanome et procédé pour leur préparation.**

(30) Priorité: **15.04.81 FR 8107596**

(43) Date de publication de la demande:
**03.11.82 Bulletin 82/44**

(45) Mention de la délivrance du brevet:
**29.01.86 Bulletin 86/05**

(84) Etats contractants désignés:
**AT BE CH DE FR GB IT LI LU NL SE**

(56) Documents cités:
**EP-A-0 023 401**
**FR-A-2 437 213**
**FR-A-2 445 149**
**FR-A-2 466 252**

**CHEMICAL ABSTRACTS, vol. 95, no. 15, 12 octobre 1981, réf. 126185u, COLUMBUS, OHIO (US)**

**NATURE, vol. 290, 12 mars 1981, Macmillan Journals Ltd H.E. BLYTHMAN et al. "Immunotoxins: hybrid molecules of monoclonal antibodies and a toxin subunit specifically kill tumour cells", pages 145-146**

(73) Titulaire: **SANOFI, société anonyme**
**40, Avenue George V**
**F-75008 Paris (FR)**

(72) Inventeur: **Jansen, Franz Klauss**
**Chemin des Fresquets ASSAS**
**F-34160 Castries (FR)**
Inventeur: **Gros, Pierre**
**18 rue des Muriers**
**F-34100 Montpellier (FR)**

(74) Mandataire: **Gillard, Marie-Louise et al**
**Cabinet Beau de Loménie 55, Rue d'Amsterdam**
**F-75008 Paris (FR)**

**EP 0 063 988 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(58) Documents cités:

**CHEMICAL ABSTRACTS, vol. 94, 1981 réf.: 132084f, page 41 COLUMBUS, OHIO (US)**

**BIOLOGICAL ABSTRACTS, vol. 71, 1981 réf.: 68145 (US)**

**BIOLOGICAL ABSTRACTS, vol. 71, 1981 réf.: 68063 (US)**

**BIOLOGICAL ABSTRACTS, vol. 71, 1981 réf.: 32655 (US)**

**BIOLOGICAL ABSTRACTS, vol. 71, 1981 réf.: 46810 (US)**

**BIOLOGICAL ABSTRACTS, vol. 71, 1981 réf.: 46871 (US)**

**BIOLOGICAL ABSTRACTS, vol. 71, 1981 réf.: 68064 (US)**

## Description

Dans les demandes antérieures FR n° 78 27 838 du 28 septembre 1978 et addition n° 79 24655 du 3 octobre 1979, publiées respectivement sous les n° 2 437 213 et 2 466 252, la demanderesse a décrit la préparation de produits anticancéreux dits conjugués obtenus par couplage par liaison covalente de la chaîne A de la Ricine à une structure protéique telle qu'un anticorps, une immunoglobuline ou un fragment d'immunoglobuline capable de reconnaître sélectivement un antigène donné à la surface des cellules porteuses que l'on veut atteindre telles que les cellules cancéreuses. La propriété principale de ces conjugués est d'être des agents cytotoxiques spécifiques des cellules cibles visées.

L'utilisation d'anticorps dirigés contre les antigènes de différentiation des cellules cancéreuses avait déjà permis d'obtenir des conjugués présentant une spécificité notable vis-à-vis des cellules cibles. La caractérisation d'antigènes associés aux cellules cancéreuses et l'obtention d'anticorps monoclonaux dirigés contre ces antigènes permettent d'envisager des conjugués présentant une spécificité accrue vis-à-vis des cellules porteuses de ces antigènes.

La présente invention a pour objet à titre de médicament un produit dit conjugué obtenu à partir de la chaîne A de la Ricine et de l'anticorps monoclonal antimélanome humain anti P97.

On entend par conjugué, une molécule mixte artificielle dans laquelle la chaîne A de la Ricine est associée, par une liaison covalente de type disulfure, à l'anticorps antimélanome humain P 97, capable de reconnaître sélectivement un antigène associé aux cellules cancéreuses.

L'obtention, de la chaîne A de Ricine pure a été décrite dans nos brevets antérieurs précités. La préparation d'anticorps monoclonaux dirigés contre des mélanomes humains a été mentionnée dans la littérature scientifique (on peut se reporter en particulier à Proceedings of the National Academy of Sciences *77* (4), 2183—7, (1980)).

Pour réaliser ledit conjugué, il est nécessaire que les protéines à coupler portent chacune au moins un atome de soufre naturellement apte ou artificiellement rendu apte à créer la liaison disulfure recherchée.

La chaîne A de Ricine présente naturellement un seul atome de soufre permettant le couplage souhaité. C'est celui de la fonction thiol du résidu de cystéine inclus dans la chaîne A et qui assurait la liaison de cette chaîne A à la chaîne B dans la toxine complète.

L'anticorps antimélanome anti P 97 ne comporte ni fonction thiol libre ni d'autres atomes de soufre susceptibles d'être utilisés pour le couplage. Il conviendra donc d'introduire artificiellement sur la molécule d'immunoglobuline un ou plusieurs atomes de soufre susceptibles d'être engagés ultérieurement dans la liaison disulfure à établir avec une ou plusieurs molécules de chaîne A de Ricine.

Selon l'invention, la préparation du conjugué est effectuée en mettant en présence la chaîne A de Ricine porteuse de son groupement SH libre avec l'anticorps dans lequel le groupement SH a été artificiellement introduit sous forme activée et notamment sous la forme d'un disulfure mixte avec un radical organique soufré convenable.

La préparation du conjugué peut être représentée par le schéma:

$$RA—SH+AC—S—S—X \rightarrow RA—S—S—AC+XSH$$

dans lequel:

. RA désigne la chaîne A de Ricine
. AC désigne l'anticorps antimélanome anti P 97
. X désigne le radical activateur.

L'anticorps substitué par un atome de soufre activé est obtenu à partir de l'anticorps lui-même, par substitution à l'aide d'un réactif lui-même porteur d'un atome de soufre activé selon le schéma:

$$AC+Y—R—S—S—X \rightarrow AC—R—S—S—X$$

dans lequel:

. AC désigne l'anticorps antimélanome anti P 97
. Y représente une fonction permettant la fixation covalente du réactif sur la protéine
. R désigne un groupement pouvant porter simultanément les substituants Y et —S—S—X
. X désigne le radical activateur.

Le groupement fonctionnel Y est une fonction capable de se lier de façon covalente avec l'une quelconque des fonctions portées par les chaînes latérales des aminoacides constitutifs de la protéine à substituer. Parmi celles-ci, les fonctions aminées terminales des radicaux lysyle contenues dans la protéine sont particulièrement indiquées. Dans ce cas, Y pourra notamment représenter:

— un groupe carboxylique qui pourra se lier aux fonctions aminées de la protéine en présence d'un agent de couplage tel qu'un carbodiimide et notamment un dérivé soluble dans l'eau comme l'éthyl-1 (diéthylamino-3 propyl)-3 carbodiimide,
— un chlorure d'acide carboxylique qui est susceptible de réagir directement avec les fonctions aminées pour les acyler,

— un ester dit "activé" tel qu'un ester d'ortho- ou para-, nitro- ou dinitro-phényle ou encore un ester de N-hydroxysuccinimide qui réagit directement avec les fonctions aminées pour les acyler,

— un anhydride interne d'un diacide carboxylique tel par exemple 1'anhydride succinique qui réagit spontanément avec les fonctions amines pour créer des liaisons amides,

— un groupement imidoester

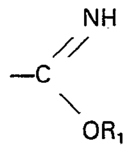

où $R_1$ est un groupe alkyle réagissant avec les groupes aminés de la protéine selon la réaction:

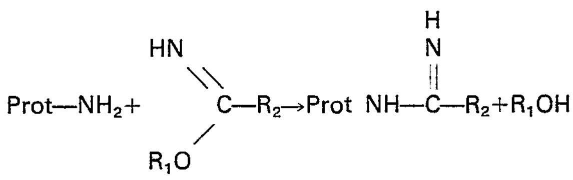

X désigne un groupement fonctionnel capable de réagir avec un radical thiol libre. En particulier, X pourra désigner un groupe pyridyl-2 ou pyridyl-4 éventuellement substitué par un ou des radicaux alkyle, halogène, carboxylique. X peut aussi désigner un groupe phényle de préférence substitué par un ou des groupes nitro- ou carboxyliques. X peut encore représenter un groupe alcoxycarbonyle tel que le groupe méthoxycarbonyle.

Le radical R désigne tout radical capable de porter simultanément les substituants Y et S—S—X. Il devra être choisi de façon à ne pas comporter de fonctions susceptibles d'interférer au cours des réactions ultérieures avec les réactifs utilisés et les produits synthétisés. En particulier, le groupe R peut être un groupe $—(CH_2)_n$ avec n compris entre 1 et 10, ou encore un groupe:

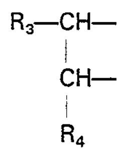

dans lequel $R_4$ désigne l'hydrogène ou un groupe alkyle ayant de 1 à 8 atomes de carbone et $R_3$ désigne un substituant inerte vis-à-vis des réactifs utilisés ultérieurement tel qu'un groupe carbamate

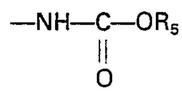

où $R_5$ désigne un groupe alkyle droit ou ramifié ayant de 1 à 5 atomes de carbone et notamnent le groupe tertiobutyle.

La réaction du composé Y—R—S—S—X avec l'immunoglobuline est effectuée en phase liquide homogéne le plus souvent dans l'eau ou une solution tampon. Lorsque la solubilité des réactifs l'exige, il est possible d'ajouter au milieu réactionnel jusqu'à 20 % en volume d'un solvent organique miscible à l'eau tel qu'un alcool et notamment le butanol tertiaire.

La réaction est effectuée à température ambiante pendant un temps variant de quelques heures à 24 heures. Après quoi, une dialyse permet d'éliminer les produits de faible masse moléculaire et en particulier les excès de réactifs. Ce procédé permet d'introduire un nombre de groupements substituants par mole de protéine compris entre 1 et 5.

En utilisant de tels composés, le couplage avec la chaîne A de Ricine est réalisé par mise en présence en solution aqueuse des deux protéines à une température ne dépassant pas 30°C pendant un temps variant de quelques heures à un jour. La solution obtenue est dialysée pour éliminer les produits de faible masse moléculaire, puis le conjugué peut être purifié par diverses méthodes connues.

L'exemple suivant permet de mieux comprendre l'invention sans en limiter la portée.

Exemple 1

Conjugué obtenu à partir d'anticorps antimélanome humain (anticorps dirigé contre l'antigène P 97) substitué par un groupe disulfure activé et la chaîne A de la Ricine.

a) Anticorps antimélanome humain (anti P 97)

Cet anticorps a été obtenu selon la méthode décrite dans Proceedings of National Academy of Sciences 1980, *77* (4), 2183—7.

Il subit une ultime purification par dialyse contre du tampon PBS (10 mM de phosphate, 140 mM de chlorure de sodium, pH: 7,4).

b) Chaîne A de la Ricine

La chaîne A de la Ricine a èté préparée et purifiée ainsi qu'il a été indiqué dans les demandes antérieures de la demanderesse (brevet n° 78 27838 et addition n° 79 24655).

c) Anticorps antimélanome humain activé

A 0,5 ml d'une solution à 20 mg/ml d'acide (pyridyl - 2 disulfanyl) - 3 propionique dans le tertiobutanol, on ajoute 0,2 ml d'une solution à 60 mg/ml d'éthyl - 1 (diméthylamino - 3 propyl) - 3 carbodiimide et laisse 3 minutes à température ambiante.

On ajoute 30 µl de la solution ainsi obtenue à 1,66 ml d'une solution d'anticorps antimélanome à 2,4 mg/ml dans le tampon PBS. On laisse l'incubation se poursuivre pendant 20 heures à 30°C.

On dialyse ensuite la solution en continu pendant 3 jours contre 21 litres de tampon PBS à 4°C. On obtient ainsi 4 mg d'anticorps activé à une concentration de 2,3 mg/ml.

Par dosage spectrophotométrique à 343 nm de la pyridine thione-2 libérée par échange avec le glutathion réduit, on constate que l'on a obtenu un anticorps portant 2,6 groupements activateurs par mole d'anticorps.

d) Conjugué

A 1,3 ml d'une solution d'anticorps activé dans le tampon PBS (concentration 2,3 mg/ml, soit 3 mg d'anticorps activé), on ajoute 0,52 ml d'une solution de chaîne A de Ricine dans le même tampon (concentration 5,8 mg/ml) et effectue l'incubation à 25°C pendant 20 heures.

On chromatographie le mélange réactionnel sur colonne de gel de Sephadex® G 100. Dans chaque fraction, on détermine la concentration en anticorps par spectrophotométrie à 280 nm et celle de la chaîne A par son pouvoir inhibiteur de la protéosynthèse mesurée sur un système acellulaire. On réunit les fractions identiques contenant le conjugué et on obtient ainsi environ 1 mg du conjugué à la concentration de 0,2 mg/ml.

Les déterminations analytiques effectuées permettent de montrer que la solution contient 50 µg/ml de chaîne A biologiquement active, soit environ 1,4 mole de chaîne A par mole d'anticorps.

Une étude effectuée par cytofluorométrie a en outre permis de montrer que l'anticorps antimélanome utilisé, l'anticorps activé correspondant et le conjugué de cet anticorps avec la chaîne A de la Ricine présentent des histogrammes de fluorescence très voisins permettant d'affirmer que l'anticorps n'a subi aucune altération importante au cours des réactions d'activation et de couplage auxquelles il a été soumis et en particulier qu'il reste capable, au sein même du conjugué, de reconnaître l'antigène P 97 contre lequel il est dirigé.

Le conjugué, selon l'invention, obtenu précédemment a été étudié en ce qui concerne ses propriétés biologiques et plus spécialement son action anticancéreuse.

1) Inhibition de la protéosynthèse

La propriété biologique fondamentale de la chaîne A de Ricine est d'inhiber la protéosynthèse cellulaire par altération de la sous-unité ribosomale 60 S.

On a utilisé ici un modèle cellulaire. Ce test mesure l'effet des substances étudiées sur l'incorporation de $^{14}$C-leucine dans les cellules cancéreuses en culture.

Les cellules utilisées appartiennent à la lignée cellulaire M 1477 issue d'un mélanome humain qui porte l'antigène P 97. Ces cellules, après trypsination, sont préincubées au moins 24 heures afin de permettre la réexpression des antigènes de surface éventuellement altérés. Après addition de la substance à étudier, on effectue une nouvelle incubation. En fin d'incubation, on procède à la mesure du taux d'incorporation de $^{14}$C-leucine par les cellules ainsi traitées.

Cette mesure est effectuée selon une technique adaptée de la technique décrite dans Journal of Biological Chemistry 1974, *249* (11), 3557—62 utilisant le traceur $^{14}$C-leucine pour la détermination du taux de protéosynthèse. La détermination de la radioactivité incorporée est effectuée ici sur les cellules entières isolées par filtration.

A partir de ces déterminations, on peut tracer les courbes effets/doses présentant en abscisse la concentration des substances étudiées et en ordonnée l'incorporation de $^{14}$C-leucine exprimée en pourcentage de l'incorporation des cellules témoins en l'absence de la substance à étudier.

On peut ainsi déterminer pour chaque substance étudiée la concentration qui inhibe 50 % de l'incorporation de $^{14}$C-leucine ou "concentration inhibitrice 50" (CI 50).

Les résultats obtenus avec le conjugué préparé dans l'exemple précédent (ITHM) sont représentés par la figure 1. Sur cette figure, sont également représentées les courbes obtenues respectivement avec la Ricine (R), la chaîne A de Ricine (AR) et un conjugué chaîne A de Ricine—anticorps anti radical dinitrophényle (DNP) (DS 10), conjugué qui ne présente aucune affinité pour les cellules testées.

On peut constater sur cette figure que le conjugué étudié (ITHM) présente une forte activité cytoxique (CI $50=5\times10^{-9}M$), environ 400 fois plus importante que celle de la chaîne A de Ricine.

Par ailleurs, le conjugué anti-DNP (DS 10) n'a pas d'effet sur les cellules M 1477 jusqu'à la plus haute concentration essayée ($10^{-6}M$). Par contre, ce même conjugué DS 10 se révèle cytotoxique acec une CI 50 voisine de $10^{-6}M$ s'il est mis en présence des mêmes cellules M 1477 préalablement rendues artificiellement porteuses de radicaux DNP. Ces deux dernières conclusions démontrent le caractère spécifique de l'activité cytotoxique du conjugué ITHM.

2) Inhibition de la formation de colonies

Les cellules en culture de mélanome M 1477 sont décollées de leur support par de la solution de Versene (tampon PBS contenant de l'acide éthylène diamine tètracétique) ou par trypsinisation. Ces cellules sont ensemencées à raison de $2\times10^4$ cellules par boîte de Pétri de 5 cm de diamètre contenant le milieu de culture suivant:

> Milieu RPMI 1640 (Mérieux) additionné de glutamine 2 mM, bicarbonate de sodium 2 g/l, 15% de sérum foetal de veau inactivé (Seromed) et d'antibiotiques (pénicilline, streptomycine et amphotéricine B).

Après 24 heures, les cellules sont traitées avec des concentrations variées du conjugué à étudier.

A titre de comparaison la même série d'expériences est effectuée avec de la Ricine, d'une part, avec la chaîne A de Ricine, d'autre part, et enfin avec un conjugué non spécifique de cette lignée cellulaire (conjugué entre la chaîne A de Ricine et une anticorps anti-DNP (DS 10)).

Après à nouveau 24 heures, le milieu de culture est éliminé et remplacé par le même milieu frais, exempt de toute substance cytotoxique.

10 à 15 jours plus tard, le nombre de colonies qui se sont développées est déterminé après coloration avec une solution de cristal violet à l'aide d'un compteur automatique de colonies (système Artek 880).

Cette méthode permet la détection d'une quantité aussi faible que 10 cellules viables par boîte ainsi que cela a pu être vérifiée en utilisant des cultures de contrôle. Il a été également démontré que la formation de colonies est strictement proportionnelle à la concentration initiale des cellules, au moins dans la limite de $10^1$ à $10^4$ cellules par ml.

Les résultats obtenus sont présentés dans la figure 2, sur laquelle on a porté, en ordonnée et en coordonnées logarithmiques, le nombre de colonies par boîte et en abscisse la concentration du produit. Les essais ont été effectués pour la Ricine (R), la chaîne A de la Ricine (AR) et le produit conjugué selon l'invention (ITHM).

Le conjugué ITHM présente une haute activité puisque la dernière cellule de mélanome est tuée à une concentration d'environ $2\times10^{-9}$ M en conjugué. Cette concentration est tout à fait comparable à celle de la Ricine ($1\times10^{-9}$ M) alors que pour la chaîne A de Ricine il faut atteindre $10^{-6}$ M pour obtenir le même effet.

On puet également noter que le conjugué DS 10 n'a aucune activité jusqu'à $2\times10^{-8}$ M la plus forte concentration à laquelle il ait été testé.

Les conclusions de cette expérience confirment entièrement celles de l'expérience d'inhibition de protéosynthèse.

Le conjugué préparé selon l'invention présente donc une forte spécificité d'action vis-à-vis des lignées cellulaires de mélanome humain. Il peut donc être utilisé en thérapeutique humaine dans le traitement des mélanomes et éventuellement d'autres affections, cancéreuses ou non, qui seraient sensibles à l'anticorps utilisé.

Ledit conjugué est conditionné pour être utilisé par voie injectable. Il peut être utilisé soit seul soit associé à un autre traitement de l'affection cancéreuse concernée et, en particulier, associé à d'autres médicaments immunodépresseurs afin de retarder et d'affaiblir la réaction immunitaire naturelle du patient vis-à-vis de la protéine étrangère à son organisme que représente le conjugué.

Visant à éliminer la totalité des cellules cancéreuses, le traitement devra être effectué avec une dose suffisante de conjugué et la durée du traitement devra être déterminée dans chaque cas en fonction du sujet et de la nature de l'affection à traiter.

**Revendications pour les états Contractants: BE, CH, DE, FR, GB, IT, LI, LU, NL, SE**

1. Médicaments utiles notamment pour le traitement des mélanomes caractérisés en ce qu'ils contiennent une substance active qui est une molécule dans laquelle la chaîne A de la Ricine est associée, par une liaison covalente de type disulfure, à l'anticorps antimélanome humain anti P 97.

2. Médicaments selon la revendication 1, caractérisés en ce qu'ils sont conditionnés en vue d'une administration par voie injectable.

3. Procédé de préparation de la substance active du médicament selon la revendication 1, caractérisé en ce que l'on fait réagir la chaîne A de la Ricine—chaîne représentée par la formule RASH—avec un dérivé de l'anticorps anti P 97 de formule AC—S—S—X dans laquelle X désigne un radical activateur et AC désigne l'anticorps anti P 97.

4. Procédé selon la revendication 3, caractérisé en ce que le radical X désigne un groupement capable

de réagir avec un radical thiol libre et notamment un groupe pyridyl-2 ou -4 éventuellement substitué, un groupe phényle ou un groupe alcoxycarbonyle.

**Revendications pour l'état Contractant: AT**

1. Procédé de préparation d'un conjugué entre la chaîne A de la Ricine et l'anticorps monoclonal antimélanome humain anti P 97, ledit conjugué étant approprié notamment pour le traitement des mélanomes, caractérisé en ce qu'il consiste à associer par une liaison covalente de type disulfure, la chaîne A de la Ricine—chaîne représentée par la formule RASH— avec un dérivé de l'anticorps anti P 97 de formule AC—S—S—X dans laquelle X désigne un radical activateur et AC désigne l'anticorps anti P 97.

2. Procédé selon la revendication 1, caractérisé en ce que le radical X désign un groupement capable de réagir avec un radical thiol libre et notamment un groupe pyridyl-2 ou -4 éventuellement substitué, un groupe phényle ou un groupe alcoxycarbonyle.

**Patentansprüche für die Vertragsstaaten: BE, CH, DE, FR, GB, IT, LI, LU, NL, SE**

1. Insbesondere zur Behandlung von Melanomen geeignete Medikamente, dadurch gekennzeichnet, daß sie eine aktive Substanz enthalten, welche ein Molekül ist, in dem die Kette A von Ricin durch eine kovalente Bindung vom Typ Disulfid an den menschlichen Antimelanom-Antikörper anti P 97 gekoppelt ist.

2. Medikamente nach Anspruch 1, dadurch gekennzeichnet, daß sie zur Verabreichung auf Injektionsweg konditioniert sind.

3. Verfahren zur Herstellung der aktiven Substanz des Medikaments nach Anspruch 1, dadurch gekennzeichnet, daß man die Kette A des Ricins—welche Kette durch die Formel RASH repräsentiert wird—mit einem Derivat des Antikörpers anti P 97 der Formel AC—S—S—X, worin, X ein Aktivatorradikal bedeutet und AC den Antikörper anti P 97 darstellt, zur Umsetzung bringt.

4. Verfahren nach Anspruch 3, dadurch gekennzeichnet, daß das Radikal X eine mit einem freien Thiolrest reaktionsfähige Gruppierung und insbesondere eine gegebenenfalls substituierte Pyridyl-2 oder -4-Gruppe, eine Phenylgruppe oder eine Alkoxycarbonylgruppe bedeutet.

**Patentansprüche für den Vertragsstaat: AT**

1. Verfahren zur Herstellung eines Konjugates zwischen der Kette A von Ricin und dem monoklonalen menschlichen Antimelanom-Antikörper anti P 97, welches Konjugat insbesondere zur Behandlung von Melanomen geeignet ist, dadurch gekennzeichnet, daß es darin besteht, daß die kette A des Ricins—welche Kette durch die Formel RASH repräsentiert wird—mit einem Derivat des Antikörpers anti P 97 der Formel AC—S—S—X, worin X ein Aktivatorradikal bedeutet und AC den Antikörper anti P 97 darstellt, durch eine kovalente Bindung vom Typ Disulfid gekoppelt wird.

2. Verfahren nach Anspruch 1, dadurch gekennzeichnet, daß das Radikal X eine mit einem freien Thiolrest reaktionsfähige Gruppierung und insbesondere eine gegebenenfalls substituierte Pyridyl-2 oder -4-Gruppe, eine Phenylgruppe oder eine Alkoxycarbonylgruppe bedeutet.

**Claims for the Contracting States: BE, CH, DE, FR, GB, IT, LI, LU, NL, SE**

1. Drugs particularly useful for the treatment of melanomae, characterized in that they contain an active substance which is a molecule in which the A chain of ricin is associated, by a covalent bound of the bisulfide type, with the human anti-melanoma antibody Anti P 97.

2. Drugs according to claim 1, characterized in that they are in a form suitable for administration by the injectable route.

3. Process for the preparation of the active substance of the drug according to claim 1, characterized in that the A chain of ricin, which chain is represented by the formula RASH, is reacted with a derivative of the antibody Anti P 97 of formula AC—S—S—X, in which X designates an activator radical, and AC designates the antibody Anti P 97.

4. Process according to claim 3, characterized in that the X radical designates a group capable of reacting with a free thiol radical and particularly an optionally substituted 2- or 4-pyridyl group, a phenyl group or an alcoxycarbonyl group.

**Claims for the Contracting State: AT**

1. Process for the preparation of a conjugate between the A chain of ricin and the human antimelanoma monoclonal antibody Anti P 97, said conjugate being particularly suitable for the treatment of melanomae, characterized in that it consists in associating by a covalent bond of disulfide type, the A chain of ricin, which chain is represented by the formula RASH, with a derivative of the antibody Anti P 97, of formula AC—S—S—X, in which X designates an activator radical and AC designates the antibody Anti P 97.

2. Process according to claim 1, characterized in that the X radical designates a group capable of reacting with a free thiol radical and particularly an optionally substituted 2- or 4-pyridyl group, a phenyl group of an alcoxycarbonyl group.

Fig. 1

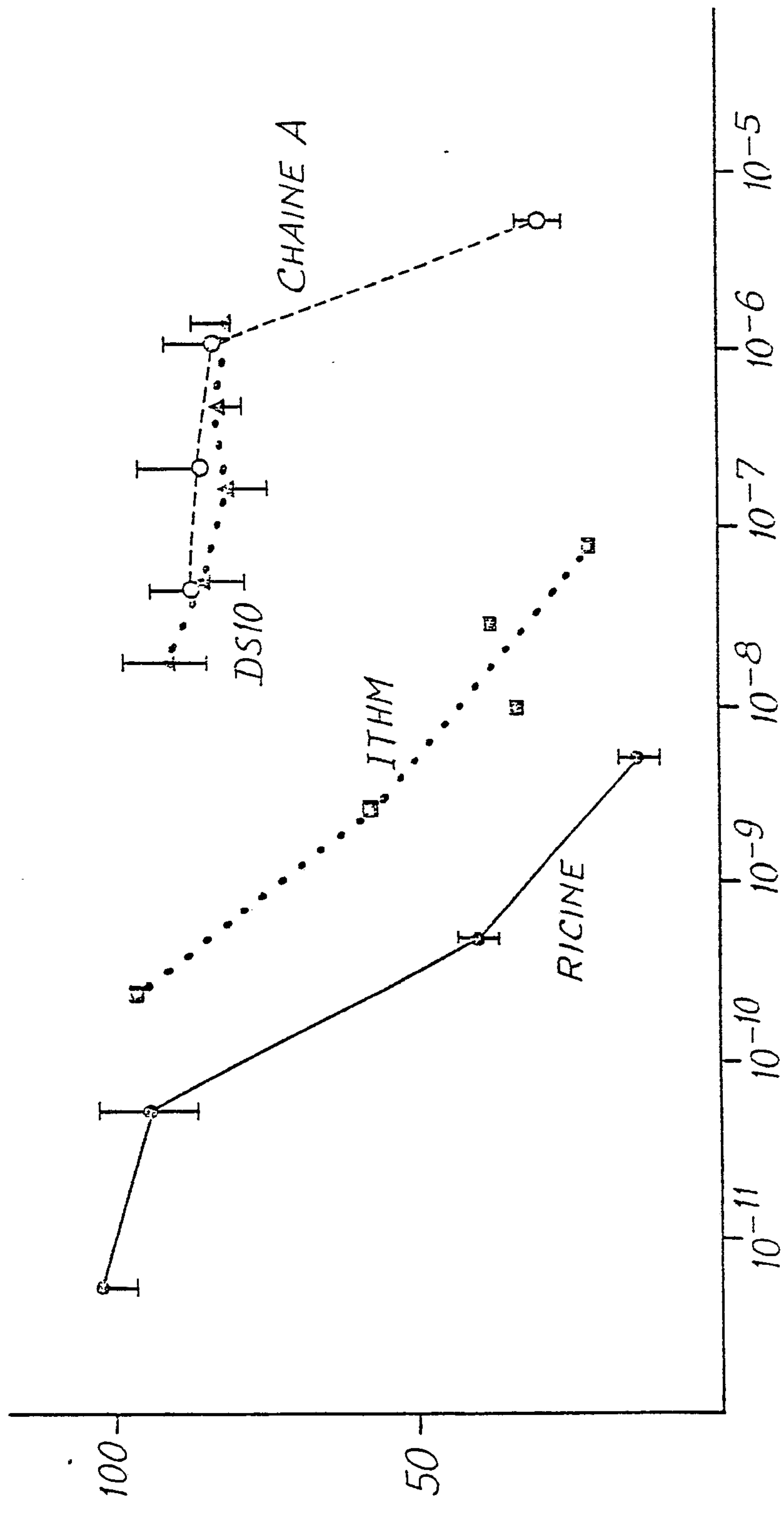

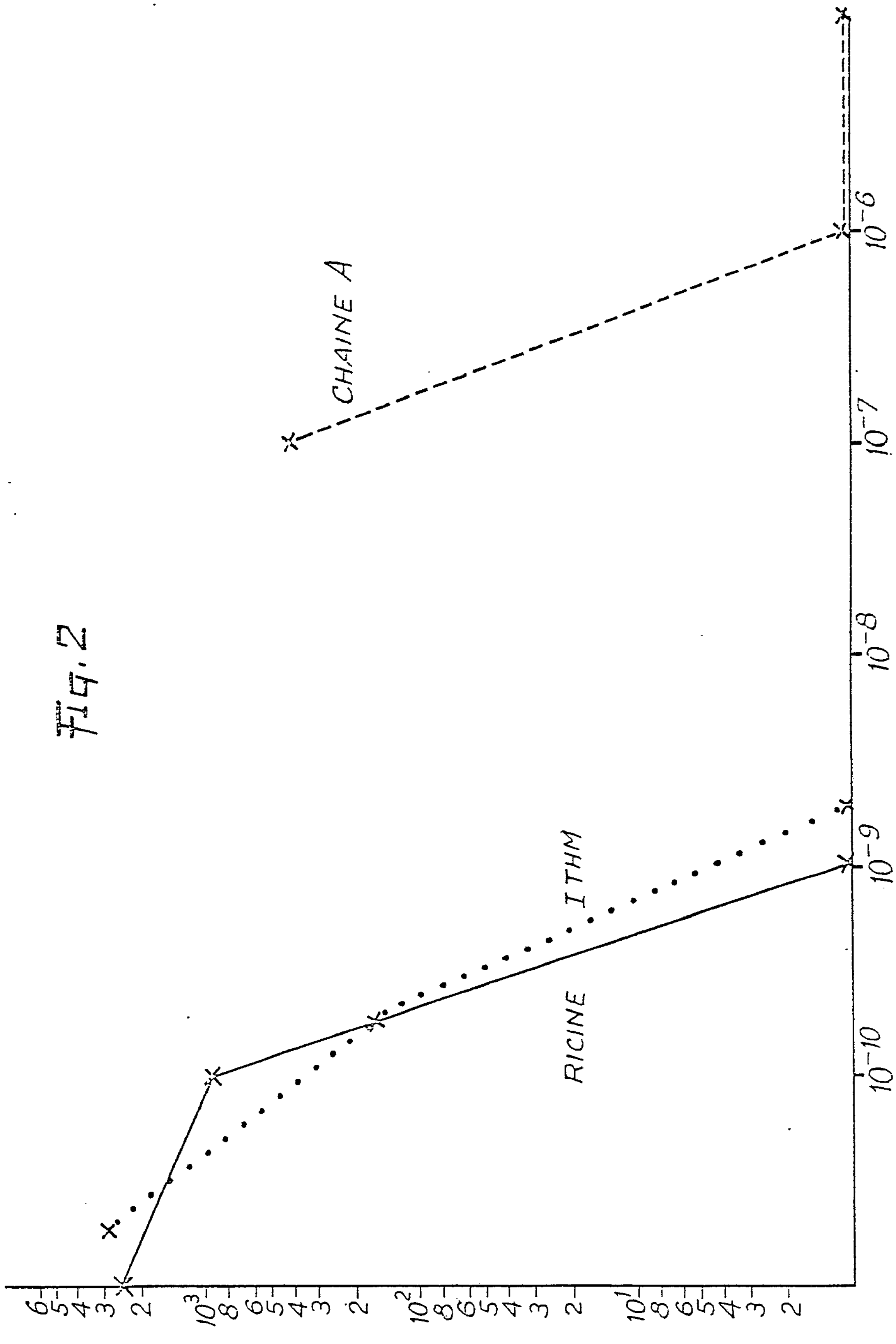


Fig. 2